Europäisches Patentamt

European Patent Office

Office européen des brevets

(19)

(11) Publication number: **0 211 257**
**A2**

(12) **EUROPEAN PATENT APPLICATION**

(21) Application number: **86109369.8**

(22) Date of filing: **09.07.86**

(51) Int. Cl.⁴: **A 61 K 9/10**
**A 61 K 9/14**

(30) Priority: **29.07.85 US 759797**

(43) Date of publication of application:
**25.02.87 Bulletin 87/9**

(84) Designated Contracting States:
**AT BE CH DE FR GB IT LI LU NL SE**

(71) Applicant: **ABBOTT LABORATORIES**
**14th Street and Sheridan Road North St**
**North Chicago Illinois 60064(US)**

(72) Inventor: **Gauthier, Robert Joseph**
**515 Beverly Place**
**Lake Forest Illinois 60045(US)**

(72) Inventor: **Levinson, Robert Saul**
**4387 Country Trail**
**Gurnee Illinois 60031(US)**

(74) Representative: **Modiano, Guido et al,**
**MODIANO, JOSIF, PISANTY & STAUB Modiano &**
**Associati Via Meravigli, 16**
**I-20123 Milan(IT)**

(54) Lyophilized emulsion compositions.

(57) Dry compositions which are easily reconstituted with water to form oil-in-water emulsions for parenteral administration are provided. The compositions can be used for parenteral nutrition or to provide parenteral emulsion vehicles for the administration of oil-soluble, water-insoluble pharmaceutical agents. The compositions are made by lyophilization of a precursor emulsion. The dry products are more convenient to manufacture, store and use than the corresponding diluted emulsions.

EP 0 211 257 A2

Croydon Printing Company Ltd.

**0211257**

LYOPHILIZED EMULSION COMPOSITIONS
AND METHOD

.TITLE MODIFIED
see front page

This invention relates to compositions for parenteral administration. More particularly, it relates to lyophilized compositions which are easily reconstituted with water to form oil-in-water emulsions for parenteral administration.

Many compositions for parenteral administration desirably take the form of oil-in-water emulsions. These include the conventional emulsions of vegetable triglycerides in water used as concentrated calorie sources in parenteral nutrition, which may also contain added amino acids and/or carbohydrates, such as dextrose or fructose. Also encompassed are oil-in-water emulsions used to deliver lipid-soluble, water-insoluble drugs via the parenteral route.

Oil-in-water emulsions for parenteral administration have certain drawbacks which make their manufacture, transportation, storage and use less convenient. These include the bulk and weight of the water used to keep the oil phase in suspension, the limited storage stability of the emulsion, the difficulty of sterilizing the emulsion without damaging the egg phospholipid emulsifiers commonly used and without causing the emulsion to break down, the limited number of concentrations in which such emulsions are available and the risk of microbial growth in the aqueous phase, which can be an especially fertile breeding ground for microorganisms if it contains added amino acids or carbohydrates. It would be desirable to provide an oil-in-water emulsion composition which is lighter and less bulky, which is storage-stable, which can be sterilized easily, which is easily prepared in any desired concentration and which eliminates the opportunity for microbial growth if it becomes contaminated.

It is an object of this invention to provide an oil-in-water emulsion for parenteral administration which is lighter and less bulky than conventional oil-in-water emulsions.

It is another object of this invention to provide an oil-in-water emulsion for parenteral administration which is storage-stable.

Yet another object of this invention is to provide an oil-in-water emulsion for parenteral administration which can be sterilized easily.

Still another object of this invention is to provide an oil-in-water emulsion for parenteral administration which can be made available in any desired concentration, limited only by the physico-chemical characteristics of the constituent materials employed.

A further object of this invention is to provide an oil-in-water emulsion for parenteral administration which eliminates the opportunity for microbial growth if it becomes contaminated.

These and other objects of the invention will be evident from the following disclosure.

## Background Art

## Disclosure of the Invention

This invention provides compositions useful in parenteral nutrition and therapy. These compositions are in lyophilized ("freeze-dried") form and contain from about 5% to about 60% pharmaceutically acceptable lipid and/or lipophilic drug, from about 0.1% to about 10% pharmaceutically acceptable emulsifier, and from about 40% to about 90% pharmaceutically acceptable solid carbohydrate by weight. These compositions, when combined with sterile, non-pyrogenic water, produce an oil-in-water emulsion suitable for parenteral administration.

When used for parenteral nutrition, the lyophilized compositions of this invention may desirably contain additional nutrients, including micronutrients, as necessary. Therefore, this invention also provides compositions which further contain one or more solutes for dissolution in the aqueous phase of the reconstituted emulsion, selected from amino acids, electrolytes, water-soluble vitamins and water-soluble trace elements, and mixtures thereof.

### Discontinuous Phase

The compositions of this invention contain from about 5% to about 60% of a component selected from pharmaceutically acceptable lipids, lipophilic drugs, and mixtures thereof. In one class of preferred embodiments of the compositions of this invention, the pharmaceutically acceptable lipid comprises a sterol or a vegetable oil. Sterols include compounds such as cholesterol, chenodeoxycholic acid, ursodeoxycholic acid, and the like. The vegetable oil, if used, is preferably selected from soybean oil triglycerides, safflower oil triglycerides, and blends thereof. Depending upon the desires of the formulator, these triglycerides may desirably comprise a medium chain ($C_6$-$C_{12}$ fatty acid chains) triglyceride fraction, which is known to be more easily metabolized by patients under metabolic or physiologic stress.

Other lipids which can be used in the compositions of this invention include synthetic and semisynthetic mono- di- and/or triglycerides, triglycerides prepared by solvent or thermal fractionation of natural, synthetic or semisynthetic triglycerides, and triglycerides prepared by interesterification and/or directed or random rearrangement.

The compositions of this invention can also be formulated so that the discontinuous phase of the reconstituted emulsion contains one or more lipophilic pharmaceutically active agents ("lipophilic drugs"), either alone or dissolved in pharmaceutically acceptable lipids. By "lipophilic" herein is meant agents which are relatively insoluble in water but soluble in one or more of the fat solvents (benzene, chloroform, acetone, ether, hexane, etc.). In particular, the lipid-containing compositions can be used as vehicles for pharmaceutically active agents having lipid component:water partition coefficients of at least 2:1.

Compositions in which the discontinuous phase of the reconstituted emulsion consists essentially of a lipophilic drug or combination of lipophilic drugs avoid the use of a separate lipid vehicle for the drug , minimize the volume of a unit dose of the drug or drugs, and reduce the risk of toxic effects which might be associated with other vehicles which would otherwise be used.

Lipophilic drugs used in the compositions of this invention are preferably selected from the group consisting of general anesthetics, local anesthetics, hypnotics, sedatives and anxiolytics, antidepressants, anticonvulsants, narcotic analgesics and narcotic antagonists, nonsteroidal antiinflammatory drugs, anticholinesterases, sympathomimetics and parasympathomimetics, ganglionic stimulating and blocking agents, neuromuscular blocking agents, antimuscarinic agents, adrenergic blocking agents, autacoids and autacoid antagonists, digitalis and digitalis congeners, diuretics and saliuretics, antibiotics and antimicrobials, antineoplastics, immunosuppressants and immunomodulators, hemoglobin and hemoglobin derivatives and polymers, hormones and hormone antagonists, and fat-soluble vitamins, and combinations thereof. These terms are used in the sense commonly employed in standard reference texts in the pharmaceutical arts, in particular, Gilman, Goodman and Gilman, The Pharmacological Basis of Therapeutics, 6th Ed. (MacMillan, New York, 1980), the disclosures of which are hereby incorporated herein by reference.

In particular, the general anesthetics can include, without limitation, diethyl ether, divinyl ether, fluroxene, methoxyflurane, halothane, etomidate, anesthetic steroids, enflurane, isoflurane, and combinations thereof.

Similarly, local anesthetics are preferably selected from cocaine, procaine, lidocaine, bupivacaine, chloroprocaine, dibucaine, etidocaine, mepivacaine, prilocaine, cyclomethycaine, hexylcaine and pramoxine, and combinations thereof.

The category of hypnotics, sedatives and anxiolytics includes the barbiturates, such as thiopental and phenobarbital; the benzodiazepines, such as diazepam and chlorazepate; the butyrophenones, such as droperidol and haloperidol; the phenothiazines, such as chlorpromazine and prochlorperazine; the thioxanthines, such as chlorprothixene; as well as the agents loxapine, molindone, chloral hydrate, chloral betaine, ethchlorvynol, ethinamate, glutethimide, meprobamate, methaqualone, methyprylon, paraldehyde, and triclofos; and combinations of any of the foregoing.

An antidepressant can be selected from imipramine, desipramine, amitriptyline, nortriptyline, doxepin, protriptyline, isocarboxazid, phenelzine and tranylcypromine, and combinations these agents.

Anticonvulsants include, without limitation, phenytoin, mephenytoin, ethotoin, primidone, carbamazepine, ethosuximide, methsuximide, phensuximide, valproic acid, valproates, trimethadione, paramethadione, and phenacemide, and combinations thereof.

Many nonsteroidal antiinflammatory drugs are preferentially soluble in lipids, including, without limitation, the salicylates, the vicinal and geminal organophosphonates, the gentisates, phenylbutazone, indomethacin, oxyphenbutazone, antipyrine, aminopyrine, apazone, acetaminophen, phenacetin, sulindac, flufenamates, mefenamates, tolmetin, ibuprofen, naproxen, fenoprofen, flurbiprofen, ketoprofen, colchicine and allopurinol, and combinations of these agents, although most are not commonly used in combination.

Compositions can also be formulated according to this invention wherein the discontinuous lipid phase contains a sympathomimetic selected from the catecholamines, such as epinephrine and isoproterenol; amphetamines, including methamphetamine and hydroxyamphetamine; and the agents ephedrine, mephentermine, metaraminol, phenylephrine, methoxamine, methoxyphenamine, metaproterenol, and terbutaline; and combinations of any of the foregoing.

Adrenergic blocking agents useful in the practice of this invention include the alpha-adrenergic blockers, such as phenoxybenzamine; beta-adrenergic blockers, such as propranolol; adrenergic neuron blockers, such as guanethidine and bretylium; and combinations of these agents.

Autacoids as used herein refers to the natural and synthetic histamines, and 5-hydroxytryptamine, and combinations thereof. Similarly, autacoid antagonists are selected from histamine ($H_1$)-receptor blockers, histamine ($H_2$)-receptor blockers, and 5-hydroxytryptamine antagonists, such as the ergot alkaloids, lysergic acid derivatives, and cyproheptadine, as well as compatible combinations of these compounds.

A variety of diuretic or saliuretic materials can be used in the practice of this invention, including those selected from the carbonic anhydrase inhibitors, such as acetazolamide; the benzothiadiazides, such as hydrochlorothiazide and methyclothiazide; and the agents ethacrynic acid, furosemide, bumetanide, muzolimine, spironolactone, triamterene, amiloride, ticrynafen and indacrynic acid, and combinations thereof.

Antimicrobial or antibiotic compounds useful in the practice of this invention include the penicillins, whether natural, or synthetic; erythromycins, sulfonamides, cephalosporins, aminoglycosides, tetracyclines, chloramphenicol, isoniazid, rifamycins, pyrazinamide, cycloserine, viomycin, lincomycins, clindamycin, spectinomycin, polymyxins, vancomycin, nystatin, amphotericin B, flucytosine, griseofulvin, amantadine, methisazone, vidarabine, idoxuridine, acyclovir and interferon, and combinations thereof.

Antineoplastic agents include a variety of classes of compounds, such as ethyleneimine derivatives, alkyl sulfonates, nitrosoureas, triazenes, folic acid analogs, pyrimidine analogs, purine analogs, vinca alkaloids, 1-asparaginase, dactinomycin, daunorubicin, doxorubicin, bleomycin, mithramycin, mitomycin, platinum coordination complexes, substituted ureas, procarbazine, mitotane and tamoxifen, and combinations thereof.

Cyclosporin is a prime example of the immunomodulators and immunosuppressants. This is a relatively new class of drugs, and few of these compounds have been approved for general clinical use. Nevertheless, the use of lipid-soluble immunomodulators and immunosuppressants in the compositions of this invention is fully contemplated.

Hemoglobin and hemoglobin derivatives and polymers includes human, animal or synthetic hemoglobin per se as well as derivatives of human, animal or synthetic hemoglobin. Derivatives include hemoglobin oligomers and polymers, liposomal hemoglobin, and hemoglobin linked to other carrier or active compounds or polymers, such as hemoglobin linked to 2-nor-2-formylpyridoxal 5'-phosphate.

Many hormones and synthetic derivatives are readily lipid soluble, particularly the synthetic steroid hormones. However, this class also includes growth hormone, prolactin, placental lactogen, luteinizing hormone, follicle stimulating hormone, thyrotropin, chorionic gonadotropin, chorionic thyrotropin, corticotropin, alpha- and beta-melanocyte stimulating hormones, beta- and gamma-lipotropins, endorphins, enkephalins, estrogens, progestins, androgens and anabolic steroids, glucocorticoids and glucocorticoid derivatives, mineralocorticoids and mineralocorticoid derivatives, insulin, glucagon, parathyroid hormone, thyroid hormone and calcitonin. Combinations of the foregoing hormones can also be used where appropriate.

The fat-soluble vitamins overlap some of the foregoing categories to a certain extent. As examples, Vitamin E is a tocopherol antioxidant, and Vitamin D (calciferol) can properly be classified as a hormone. The fat soluble vitamins also encompass the carotenes, including Vitamin A and precursors; synthetic calciferols and tocopherols, Vitamin K, including the menaquinones, phytonadione and menadione, and combinations thereof.

In these preferred compositions, the emulsifier comprises nontoxic phospholipids. Most preferably, the emulsifier comprises egg phosphatides.

Because of the high particle surface area offered by the particulate products of this invention, the ingredients may be prone to oxidation, particularly if the content of unsaturated fatty acid moieties is high. In such cases, the compositions will preferably also contain a tocopherol or other lipophilic antioxidant.

## Aqueous Continuous Phase

Also according to the desires of the formulator, the compositions of this invention can be formulated such that the aqueous continuous phase of the reconstituted emulsion comprises one or more solutes selected from amino acids, electrolytes, water-soluble vitamins and water-soluble trace elements, and mixtures thereof.

The amino acids administered in the compositions of this invention can include, without limitation, leucine, isoleucine, lysine, methionine, phenylalanine, threonine, tryptophan, valine, alanine, arginine, histidine, proline, serine, tyrosine, glycine, taurine and carnitine, as the L-, D-, or racemic forms, as well as nontoxic salts and esters thereof. Most preferred are the L-acids, and their nontoxic salts and esters. Of particular value in formulating the compositions of this invention is the amino acid carnitine, which has been reported to facilitate the metabolism of lipids and other amino acids. When added to the compositions of this invention, the carnitine is preferably added in an amount sufficient to enhance metabolism of the lipids in the discontinuous phase, but can be added in am amount sufficient to enhance the metabolism of other amino acids in the continuous phase.

The amino acids added to the compositions of this invention also preferably comprise branched chain amino acids, which are reported to facilitate normalization of plasma amino acid levels, particularly in debilitated or liver-damaged patients.

According to the desires of the formulator, electrolytes added to the compositions of this invention can include, without limitation, sodium, potassium, magnesium, calcium, lithium, ammonium, phosphorus, chloride, acetate, sulfate, carbonate, phosphate, lactate, gluconate and lactobionate in pharmaceutically acceptable amounts. In measuring or calculating the electrolyte content of the solutions, the electrolyte equivalents of any amino acids included in the formulation should also be included.

The water-soluble vitamins useful in the compositions of this invention are well known and include the B-vitamins, Vitamin C, and minor vitamins such as bioflavonoids and biotin. The water-soluble trace elements include chromium, selenium, copper, calcium, zinc, magnesium and iron.

### Carbohydrates

The compositions of this invention employ a carbohydrate carrier material which can be lyophilized to dryness and which should be selected not to interfere with the other components of the composition. Carbohydrates useful in formulating the compositions of this invention include monosaccharides, disaccharides, oligosaccharides, and polysaccharides. The monosaccharides as defined herein include, without limitation, the sugars dextrose, fructose and galactose, and the sugar alcohols mannitol, xylitol, inositol and sorbitol. The disaccharides include, without limitation, sucrose, lactose and maltose. Oligosaccharides include polymers of the above-described monosaccharide sugars containing from 3 to 6 units. Polysaccharides include the well-known dextrans, typically having molecular weights of from 40,000 to 70,000, and such materials as hydroxyethyl starch.

"Pharmaceutically acceptable" is used herein to refer to those materials which are, within the scope of sound medical judgment, suitable for use in contact with the tissues of humans and lower animals without undue toxicity, irritation, allergic response, and the like, commensurate with a reasonable benefit/risk ratio, and effective for their intended use in the composition.

## Physical Properties

Although they contain only conventional emulsifiers employed at their usual levels, the compositions of this invention are highly storage stable, since they can be kept for extended periods in dry form.

Compositions can also be formulated according to this invention to provide reconstituted microemulsions which are substantially transparent, i.e., have a turbidity of less than 30 Nephelometric Turbidity Units (NTU) when measured by published standard methods. Transparency is imparted, in general, by preparation of the desired composition in microemulsion form. The disperse or discontinuous phase particles in microemulsions have mean particle diameters of less than 0.1 micron, and a particle size distribution such that less than 1% of the particles in the discontinuous or disperse phase have diameters greater than 0.1 micron. Although the preparation of such emulsions per se forms no part of this invention, microemulsions useful in preparing the compositions of this invention can be made according to our copending U.S. Patent Application, Serial Number 759,796 , Filed July 29, 1985, Attorney's Docket Number 4276, the disclosures of which are hereby incorporated herein by reference. In some instances, the addition of optional ingredients as provided herein (e.g., carotenes in the lipid phase or riboflavin in the aqueous phase) will impart a color or haze to the composition.

### Delivery Systems

The compositions of this invention are formulated for parenteral administration. By parenteral administration is meant routes of administration other than enteral and topical, usually by injection, and includes, without limitation, intravenous, intraarterial, intrathecal, perineural, intracardiac, intraperitoneal, transtracheal, intramuscular, subcutaneous, subcuticular, intraarticular, subcapsular, subarachnoid, and epidural injection. In designing parenteral delivery systems for the lyophilized compositions of this invention, provision must be made for separating the dry composition as provided herein from the intended aqueous pharmaceutical diluent. Accordingly, it may be preferred to simply package the lyophilized compositions separately, and reconstitute them at the time of use with commercially available Water for Injection, U.S.P.; 0.9% Sodium Chloride Injection, U.S.P.; 5% Dextrose Injection, U.S.P.; or a similar readily available pharmaceutical diluent. In the interest of convenience, dual chambered containers and associated administration devices and the like are useful and preferred in storing and delivering the compositions of this invention in conjunction with the desired aqueous diluent. Within this limitation, however, wide latitude exists in the design of appropriate dosage delivery devices.

Delivery systems for parenteral administration are usually divided into two categories: large volume parenteral (LVP) delivery systems and small volume parenteral (SVP) delivery systems. LVP delivery systems are commonly used when a large volume of fluid is required or desired to be given directly by the intravenous route, while small volume parenterals are commonly used to provide single or multiple injections via any of the above-mentioned routes. Also, a drug provided in an SVP delivery system can be added to an LVP delivery system to provide diluted, but continuous, intravenous administration of the drug.

LVP delivery systems commonly provide volumes of from 100 ml (which is considered an LVP dose for pediatric use) to 3000 ml. These volumes of fluid are available both in glass bottles and rigid, semi-rigid, and flexible containers fabricated from a variety of polymers and polymer combinations well known to the art. Each of these forms is suitable for use in the practice of the present invention, provided the usual safety and other standards for parenteral solution containers are met.

SVP delivery systems are also available in a variety of forms, depending upon the specific drug involved, its intended use, and the volume of fluid to be administered. SVP delivery systems typically contain from 0.1 ml to 100 ml of fluid. These delivery systems include glass and polymeric ampuls; glass and polymeric "fliptop" vials, which are intended for use in filling syringes; pressurized and unpressurized "pintop" vials, which incorporate a transfer cannula and are intended for use in adding the contents to LVP delivery systems; and a number of styles of prefilled syringes. Each is suitable for use in the practice of this invention, subject only to the usual criteria which dictate selection of such delivery systems, and the above-mentioned considerations regarding separation of the active dry component from the diluent until reconstitution.

## Manufacture

The compositions of this invention are made, in general, by preparing an emulsion in liquid form having the composition of the reconstituted emulsion desired or some multiple of concentration of the reconstituted emulsion desired. This source emulsion is then combined with a sugar capable of being dehydrated to totally dry form by sublimation of the water in the solution. The resulting liquid is then sprayed as fine droplets into a bath of boiling fluid having a boiling point below -20°C.

In general, any fluid which has a boiling point below about -20°C can be used in this process, so long as it is not chemically incompatible with the constituents of the emulsion. Especially preferred for their relative density and inertness are the fluorocarbon refrigerants.

Upon exposure to the cold fluid, the droplets rapidly freeze in the form of fine particles. The size of the droplets/particles formed in this process is not critical, since it bears no relation to the droplet size of the disperse phase in the reconstituted emulsion. However, the droplets/particles should not be so large as to preclude convenient dispensing and measuring of desired quantities of the lyophilized product, nor so small that electostatic forces will make handling difficult. In general, the droplets/particles will have an average diameter of from 0.1 mm to about 2 mm. The particulate form increases the surface area of the lyophilized product, so that it can be rapidly reconstituted in water or other aqueous diluent.

The source emulsion concentration and the fluid are preferably selected such that the frozen particles will have a density different from that of the fluid, so that they can then be harvested by known density separation methods and dehydrated under conventional lyophilization conditions of vacuum and temperature. Substantially all of the water is removed from the particles as the ice therein sublimes under the conditions of lyophilization. The resulting dry, free-flowing particles are then collected for subsequent processing, such as sterilization or packaging. The foregoing principles are further discussed in U.S. Patents 3,721,725 "Process for Preparing Powder Blends" and 3,932,943 "Method of Preparation of Lyophilized Biological Products", the disclosures of which are hereby incorporated by reference.

## Industrial Applicability

The following examples illustrate the practice of this invention, but are not intended to be limitative thereof. All percentages herein are by weight unless otherwise indicated.

## Example 1

An emulsion mixture consisting of the following formulation (w/v) was prepared by conventional emulsification:

| | |
|---|---|
| Safflower Oil | 5% |
| Soybean Oil | 5% |
| Egg Phosphatides | 1.2% |
| Glycerin | 2.5% |
| Water for Injection | qs.ad 1000.0 ml |

The emulsion so produced exhibited an arithmetic mean globule size of 0.40 micron (SEM $\pm$ 0.07). Anhydrous Dextrose, 50% w/v, was added to the emulsion. The mixture was then sprayed onto a moving bath of fluorocarbon (Freon-12$^{TM}$) refrigerant at a temperature below -20° C. The frozen droplets were harvested with a screen paddle and transferred while still frozen to a precooled FTS Systems Model TDS-3 lyophilizer with the following lyophilization conditions maintained.

| | | |
|---|---|---|
| 0 Time | : | -30° C. |
| 24 Hours | : | -20° C. |
| 48 Hours | : | -5° C. |
| 60 Hours | : | +15° C. |
| 72 Hours | : | OUT |

The resulting free-flowing lyophilized powdery material was removed from the lyophilizer and appropriately packaged. The reconstitution of the emulsion mixture by adding Water for Injection, USP resulted in instantaneous reconstitution of the emulsion having an arithmetic mean globule size of 0.48 to 0.70 micron.

### Example 2

To the emulsion mixture of Example 1 were added crystalline amino acids and dextrose to provide a parenteral solution having the following composition (w/v):

| | |
|---|---|
| Safflower Oil | 5% |
| Soybean Oil | 5% |
| Egg Phosphatides | 1.2% |
| Glycerin | 2.5% |
| Crystalline Amino Acids | 7.5% |
| Dextrose | 50% |
| Water for Injection | qs.ad 1000.0 ml |

The emulsion, using the same lipid composition as Example 1, had the same mean globule size. The mixture was sprayed onto a moving bath of fluorocarbon (Freon-12$^{TM}$) refrigerant at a temperature below -20° C. The frozen droplets were harvested and lyophilized using the same equipment and conditions as in Example 1. The free-flowing lyophilized powder-like material was removed from the lyophilizer and appropriately packaged. The product was later reconstituted by adding Water for Injection, USP to produce an emulsion with an arithmetic mean globule size of 0.48 to 0.70 micron.

### Example 3

An emulsion mixture having the following formulation (w/v) was prepared as in Example 2:

| | |
|---|---|
| Safflower Oil | 10% |
| Egg Phosphatides | 1.2% |
| Glycerin | 2.5% |
| Trehalose | 20% |
| Water for Injection | qs.ad 1000.0 ml |

The emulsion had the same arithmetic mean globule size of 0.40 micron ($\pm$ 0.07). The mixture was proglobule size of 0.40 micron ($\pm$ 0.07). The mixture was processed as in Examples 1 and 2.

The free-flowing lyophilized powder-like material was removed from the lyophilizer and appropriately packaged. When reconstituted by adding Water for Injection, USP, the emulsion also had an arithmetic mean globule size of 0.48 to 0.70 micron.

### Example 4

A lyophilized product within the scope of this invention can be prepared by lyophilization of a parenteral emulsion having the following composition (w/v), using the methods of Examples 1-3:

| | |
|---|---|
| Soybean Oil | 10% |
| Egg Phosphatides | 1.2% |
| Glycerin | 2.5% |
| Trehalose | 20% |
| Water for Injection | qs.ad 1000.0 ml |

## Example 5

An emulsion having the following composition (w/v) and having a mean particle size of 0.40 micron ($\pm$ 0.07) was prepared by conventional emulsification:

| | |
|---|---|
| Safflower Oil | 5% |
| Soybean Oil | 5% |
| Egg Phosphatides | 1.2% |
| Glycerin | 2.5% |
| Dextrose | 10% |
| Trehalose | 10% |
| Water for Injection | qs.ad 1000.0 ml |

The mixture was processed as using the same equipment and conditions as in Examples 1-3. The free-flowing lyophilized powder-like material was removed from the lyophilizer and appropriately packaged. The reconstitution of the emulsion mixture by adding Water for Injection, USP resulted in instantaneous reconstitution of the emulsion with an arithmetic mean globule size of 0.48 to 0.70 micron.

## Example 6

A microemulsion mixture is prepared using a Microfluidics (Newton, Mass.) Microfluidizer[TM] Model 110A. The microemulsion formulation (w/v) was as follows:

| | |
|---|---|
| Safflower Oil | 5% |
| Soybean Oil | 5% |
| Egg Phosphatides | 1.2% |
| Glycerin | 2.5% |
| Dextrose | 50% |
| Water for Injection | qs.ad 1000.0 ml |

Processing the microemulsion in the Microfluidizer device is continued until the mean globule size is found to be below 125 nanometers (0.125 micron).

The microemulsion so produced is sprayed onto a moving bath of fluorocarbon (Freon-12[TM]) refrigerant at a temperature of below -20° C. The frozen droplets are harvested with a screen paddle and transferred while still frozen to a precooled FTS Systems (Model TDS-3) lyophilizer with the following lyophilization conditions maintained:

```
 0 Time   :   -30° C.
24 Hours  :   -20° C.
48 Hours  :    -5° C.
60 Hours  :   +15° C.
72 Hours  :    OUT
```

The free-flowing lyophilized powder-like material is within the scope of this invention. The powder can be removed from the lyophilizer and appropriately packaged for later reconstitution with Water for Injection.

### Example 7

An emulsion having the following formulation is prepared by conventional emulsification to an arithmetic mean globule size of 0.40 micron:

| | |
|---|---|
| Lidocaine, USP | 1% |
| Safflower Oil | 5% |
| Soybean Oil | 5% |
| Egg Phosphatides | 1.2% |
| Glycerin | 2.5% |
| Dextrose | 20% |
| Water for Injection | qs.ad 1000.0 ml |

The mixture is then processed as in Examples 1-3. The free-flowing lyophilized powder-like material is within the scope of this invention. It can be removed from the lyophilizer and appropriately packaged for reconstitution by adding Water for Injection, USP.

### Example 8

A microemulsion mixture having the following composition is prepared and lyophilized as in Example 6:

| | |
|---|---|
| Lidocaine, USP | 1% |
| Soybean Oil | 5% |
| Safflower Oil | 5% |
| Egg Phosphatides | 1.2% |
| Glycerin | 2.5% |
| Dextrose | 20% |
| Water for Injection | qs.ad 1000.0 ml |

The resulting lyophilized product is within the scope of this invention.

CLAIMS:

1. A lyophilized composition comprising:

from about 5% to about 60% of a component selected from pharmaceutically acceptable lipids, lipophilic drugs, and mixtures thereof;

from about 0.1% to about 10% of a pharmaceutically acceptable emulsifier; and

from about 40% to about 90% pharmaceutically acceptable solid carbohydrate by weight and which, when combined with sterile, non-pyrogenic water, produces an oil-in-water emulsion suitable for parenteral administration.

2. A composition according to Claim 1 wherein the pharmaceutically acceptable lipid comprises a vegetable oil.

3. A composition according to Claim 4 wherein the vegetable oil further comprises a medium chain triglyceride fraction.

4. A composition according to Claim 1 wherein the emulsifier is a phospholipid emulsifier.

5. A composition according to Claim 6 wherein the phospholipid emulsifier comprises egg phosphatides.

6. A composition according to Claim 1 which further comprises a solute component selected from amino acids, electrolytes, water-soluble vitamins and water-soluble trace elements, and mixtures thereof.

7. A composition according to Claim 10 wherein the amino acids comprise branched chain amino acids.

8. A composition according to Claim 1 wherein the discontinuous phase comprises a lipophilic drug.

9. A composition according to Claim 14 wherein the lipophilic drug has a discontinuous phase:continuous phase partition coefficient of at least 2:1.

10. A composition according to Claim 14 wherein the lipophilic drug is selected from the group consisting of general anesthetics, local anesthetics, hypnotics, sedatives and anxiolytics, antidepressants, anticonvulsants, narcotic analgesics and narcotic antagonists, nonsteroidal antiinflammatory drugs, anticholinesterases, sympathomimetics and parasympathomimetics, ganglionic stimulating and blocking agents, neuromuscular blocking agents, antimuscarinic agents, adrenergic blocking agents, autacoids and autacoid antagonists, digitalis and digitalis congeners, diuretics and saliuretics, antibiotics and antimicrobials, antineoplastics, immunosuppressants and immunomodulators, hemoglobin and hemoglobin derivatives and polymers thereof, hormones and hormone antagonists, and fat-soluble vitamins, and combinations thereof.